Europäisches Patentamt

European Patent Office

Office européen des brevets

(11) Veröffentlichungsnummer: **0 222 302 B1**

## EUROPÄISCHE PATENTSCHRIFT

(45) Veröffentlichungstag der Patentschrift: **27.05.92**

(51) Int. Cl.⁵: **C12P 19/10**

(21) Anmeldenummer: **86115267.6**

(22) Anmeldetag: **04.11.86**

(54) **Aureobasidium-pullulans-Stamm, Herstellungsverfahren und Verwendung.**

(30) Priorität: **05.11.85 DE 3539180**

(43) Veröffentlichungstag der Anmeldung:
**20.05.87 Patentblatt 87/21**

(45) Bekanntmachung des Hinweises auf die
Patenterteilung:
**27.05.92 Patentblatt 92/22**

(84) Benannte Vertragsstaaten:
**AT CH DE FR GB IT LI NL SE**

(56) Entgegenhaltungen:

CHEMICAL ABSTRACTS, Band 100, Nr. 3, 16
Januar 1984, Columbus, OH (US);
L.A.KOSSIOR et al., Seite 184, Nr. 19055b

CHEMICAL ABSTRACTS, Band 98, Nr. 9, 28
Februar 1983, Columbus, OH (US);
N.P.ELINOV et al., Seite 284, Nr. 67978s

CHEMICAL ABSTRACTS, Band 88, Nr. 5, 30
Januar 1978, Columbus, OH (US); P.J.KELLY
et al., Seite 239, Nr. 34389r
(73) Patentinhaber: **Consortium für elektrochemische Industrie GmbH
Zielstattstrasse 20
W-8000 München 70(DE)**

(72) Erfinder: **Böck, August, Prof.Dr.
Lindenstr. 10
W-8085 Geltendorf(DE)**
Erfinder: **Lechner, Konrad
Turnerstr. 33
W-8000 München 82(DE)**
Erfinder: **Huber, Otto, Dr.
Breitbrunner Str. 17
W-8000 München 70(DE)**

## Beschreibung

Die Erfindung betrifft einen Aureobasidium-pullulans-Stamm, der Pullulan, aber praktisch kein Melanin produziert, sowie ein Verfahren zu seiner Herstellung und seine Verwendung.

Es ist bekannt, daß Stämme der Gattung Aureobasidium Pullulan produzieren. Pullulan ist ein Polysaccharid mit Maltotrioseeinheiten (alpha1--4-Bindungen), die folgendermaßen verknüpft sind: alpha1--6; vgl. Bernier in Can. J. Microbiol., 4 (1958) 195 - 204 und Bender & Lehnmann & Wallenfels in Biochim. Biophys. Acta, 36 (1959) 309 - 316.

Das Polysaccharid Pullulan findet verschiedene Verwendung, beispielsweise

(1) zur Herstellung transparenter Filme, die für Kohlendioxid, jedoch nicht für Sauerstoff durchlässig sind,

(2) als Flockungsmittel und

(3) als Dextranersatz in Infusionsflüssigkeiten.

Stämme der Gattung Aureobasidium bilden während der Kultivierung ein grün-schwarzes Pigment (Melanin). Dieses Melanin kann bei der üblichen Extraktion von Pullulan nicht abgetrennt werden. Es ist daher eine Teilaufgabe der Erfindung, Aureobasidium-pullulans-Stämme vorzusehen, die gegenüber dem Stand der Technik in vermindertem Umfang Melanin produzieren. Gemäß weiteren Teilaufgaben der Erfindung sollen ein Verfahren zur Herstellung derartiger Stämme sowie die Verwendung dieser Stämme zur Herstellung von Pullulan vorgesehen werden.

Demgemäß betrifft die Erfindung einen Aureobasidium-pullulans-Stamm, der Pullulan, aber eine verminderte Melaninmenge produziert, und der dadurch erhältlich ist, daß man

(a) einen A.-pullulans-Stamm, der Pullulan und Melanin produziert, in an sich bekannter Weise mutierenden Bedingungen aussetzt,

(b) danach den Stamm, der den mutierenden Bedingungen ausgesetzt worden ist, in an sich bekannter Weise kultiviert und

(c) einen mutierten A.-pullulans-Stamm (in Form von einer oder mehreren Kolonien) selektiert, und davon abgeleitete Stämme, wobei der selektierte Stamm und die abgeleiteten Stämme im Vergleich zum Ausgangsstamm weniger oder nicht pigmentiert sind.

Den mutierten Stamm kann man beispielsweise auf einem Agarplatten-Kulturmedium kultivieren. Die selektierten Kolonien kann man in Flüssigkultur daraufhin prüfen, ob sie noch Pullulan in befriedigender Ausbeute produzieren.

Erfindungsgemäße A.-pullulans-Stämme sind dadurch erhältlich, daß man durch Bestrahlung mit UV-Licht oder chemische Behandlung mutiert, insbesondere durch Behandlung mit Ethylmethansulfonat.

Die Melaninbildung benötigt einige Zeit. Um sich ein Urteil über die Melaninbildung oder die ausbleibende Melaninbildung von Kulturen machen zu können, ist es vorteilhaft, wenn während dieser Zeitspanne die einzelnen Kolonien nicht zu einem Zellrasen zusammenwachsen. Dazu kann man bei der Stufe (b) in einem Temperaturbereich kultivieren, bei dem keine oder eine geringe Zellvermehrung, jedoch noch Melaninbildung stattfindet, insbesondere bei einer Temperatur im Bereich von 2 bis 13 und vorzugsweise 4 bis 10 °C.

Als Ausgangsstamm kann man ATCC 9348 verwenden.

Ein Mutationsprodukt dieses bekannten Stammes ist der A.-pullulans-Stamm P 56, der bei der folgenden Hinterlegungsstelle hinterlegt und die folgende Hinterlegungsnummer erhalten hat: Deutsche Sammlung für Mikroorganismen, DSM 3562. P 56 unterscheidet sich von ATCC 9348 mykologisch dadurch, daß der erfindungsgemäße Stamm kein Melanin bildet und die Pullulanbildung um 50 % und mehr als beim Ausgangsstamm erhöht ist.

Erfindungsgemäße A.-pullulans-Stämme lassen sich dadurch herstellen, daß man die vorstehend genannten Stufen (a) bis (c) gegebenenfalls mit den spezielleren vorstehend genannten Maßnahmen durchführt.

Pullulan kann man dadurch gewinnen, daß man einen erfindungsgemäßen A.-pullulans-Stamm in einem Kulturmedium kultiviert, Pullulan produzieren läßt und das gebildete Pullulan vom Kulturmedium abtrennt. Das Kulturmedium soll Kohlenstoffquellen enthalten, die der Stamm verwerten kann (beispielsweise Glukose, Saccharose, Maltose oder Xylose), sowie Hefeextrakt und anorganische Salze, die für das Zellwachstum wesentlich sind. Üblicherweise kultiviert man unter aeroben Bedingungen als Schüttelkultur oder Submerskultur unter Belüften bei einer Temperatur von beispielsweise 20 bis 30 °C und einem pH-Wert von beispielsweise 2,5 bis 6.

Nachstehend wird die Erfindung durch Beispiele näher erläutert.

### Beispiel 1

Man kultiviert einen im Handel erhältlichen A.-pullulans-Stamm (beispielsweise ATCC 9348) in einem Kulturmedium nach Le Duy mit einem Gehalt an: 5 g/l $K_2HPO_4$, 1 g/l NaCl, 0,6 g/l $(NH_4)_2SO_4$, 0,2 g/l $MgSO_4$, 0,4 g/l Hefeextrakt und 30 g/l Saccharose, wobei der pH-Wert mit HCl auf 5,5 eingestellt wird.

Der Stamm wird 24 h lang in dem genannten Medium kultiviert. Danach trennt man das Mycel durch Zentrifugieren bei 6000 x g in 5 min ab. Der Überstand wird erneut bei 17000 x g 10 min lang zentrifugiert. Die beim Zentrifugieren angefallenen Pellets wäscht man mit physiologischer Kochsalzlösung, wonach man die Zelldichte auf $1 \times 10^7$ Zellen/ml einstellt. Danach bestrahlt man die Suspension mit UV-Licht (354 nm; 1400 $\mu$W/cm$^2$) 7 bis 8 min lang.

Die Suspension (0,1 ml) platiert man 2 bis 3 d lang auf einer Agar-Kulturplatte (Extrakt von 300 g Kartoffeln, 20 g Glukose und 15 g Agar/l). Die Platten läßt man 8 d lang bei 4 °C stehen. Danach selektiert man unpigmentierte und praktisch unpigmentierte Kolonien. Die selektierten Kolonien überprüft man daraufhin, ob sie noch in befriedigendem Maße Pullulan produzieren.

Beispiel 2

Man stellt ein Kulturmedium mit folgenden Bestandteilen her: 1 l destilliertes Wasser, 5 g $K_2HPO_4$, 1 g NaCl, 0,6 g $(NH_4)_2SO_4$, 0,2 g $MgSO_4$ x 7 $H_2O$, 0,4 g Hefeextrakt und 30 g Kohlenstoffquellen (vgl. vorstehend).

100 ml des Kulturmediums werden mit HCl auf einen pH-Wert von 5,5 eingestellt.

Danach gibt man das Kulturmedium in einen 500-ml-Erlenmeyerkolben und sterilisiert. Danach inokuliert man das Kulturmedium mit dem erfindungsgemäßen Stamm P 56 , der gemäß Beispiel 1 hergestellt wurde. Man kultiviert bei 26 °C unter Rühren (200 U/min).

Nach 7-tägigem Kultivieren zentrifugiert man die Kultur bei 27000 x g 15 min lang. Zum Überstand gibt man das doppelte Volumen an 96-proz. Ethanol, mischt und zentrifugiert wieder bei 1000 x g 10 min lang, wobei sich weißes Pullulan abtrennt. Das gesammelte Produkt wird gewaschen, gemahlen, wieder in Wasser aufgelöst, erneut mit Ethanol gefällt und danach getrocknet.

Zur Identitätsprüfung kann man das erhaltene Produkt einer $^{13}$C-NMR-Analyse sowie einer Hochleistungsflüssigchromatographie (HPLC) unterwerfen.

Vergleichsbeispiel 1

Beispiel 2 wird mit der Ausnahme wiederholt, daß man einen A.-pullulans-Stamm des Handels (beispielsweise ATCC 9348) verwendet. Das erhaltene Pullulan besitzt eine intensive grün-schwarze Färbung.

**Patentansprüche**

1. Aureobasidium-pullulans-Stamm, der Fullulan, aber eine verminderte Melaninmenge produziert, dadurch **erhältlich,** daß man

    (a) Aureobasidium pullulans Stamm ATCC 9348 in an sich bekannter Weise mutierenden Bedingungen aussetzt,

    (b) danach den Stamm in an sich bekannter Weise kultiviert und

    (c) einen mutierten A.-pullulans-Stamm (in Form von einer oder menreren Kolonien) selektiert,

wobei der selektierte Stamm im Vergleich zum Ausgangsstamm weniger oder nicht pigmentiert ist.

2. Stamm nach Anspruch 1, dadurch **erhältlich,** daß man durch Bestrahlung mit UV-Licht oder chemische Behandlung mutiert, insbesondere durch Behandlung mit Ethylmethansulfonat.

3. Stamm nach Anspruch 1 oder 2, dadurch **erhältlich,** daß man bei der Stufe (b) in einem Temperaturbereich kultiviert, in dem keine oder eine geringe Zellvermehrung, jedoch noch Melaninbildung stattfindet, insbesondere bei einer Temperatur im Bereich 2 bis 13, vorzugsweise 4 bis 10 °C.

4. Aureobasidium-pullulans-Stamm DSM 3562

5. Verfahren zur Herstellung eines A.-pullulans-Stammes gemäß einem der Ansprüche 1 bis 4, dadurch **gekennzeichnet,** daß man die Stufen (a) bis (c) gemäß Anspruch 1 durchführt.

6. Verfahren nach Anspruch 5, dadurch **gekennzeichnet,** daß man die Maßnahmen gemäß einem der Ansprüche 2 und 3 durchführt.

7. Verwendung eines A.-pullulans-Stammes gemäß einem der Ansprüche 1 bis 4 zur Herstellung von Pullulan.

**Claims**

1. Aureobasidium pullulans strain which produces pullulan but a reduced quantity of melanin, which can be obtained by

    (a) exposing Aureobasidium pullulans strain ATCC 9348 to mutating conditions in a manner known per se,

    (b) then cultivating the strain in a manner known per se, and

    (c) selecting a mutated A. pullulans strain (in the form of one or more colonies),

the pigmentation of the selected strain being less than that of the initial strain or zero.

2. Strain according to Claim 1, which can be obtained by mutation by irradiation with UV light or chemical treatment, in particular by treatment with ethyl methanesulfonate.

**3.** Strain according to Claim 1 or 2, which can be obtained by cultivation, in stage (b), in a temperature range at which little or no cell multiplication, but still the formation of melanin, takes place, in particular at a temperature in the range 2 to 13, preferably 4 to 10, °C.

**4.** Aureobasidium pullulans strain DSM 3562.

**5.** Process for the preparation of an A. pullulans strain according to one of Claims 1 to 4, characterised in that stages (a) to (c) are carried out according to Claim 1.

**6.** Process according to Claim 5, characterised in that the measures according to one of Claims 2 and 3 are carried out.

**7.** Use of an A. pullulans strain according to one of Claims 1 to 4 for the preparation of pullulan.

**Revendications**

**1.** Souche d'Aureobasidium pullulans produisant du pullulane mais une quantité réduite de mélanine, souche que l'on peut obtenir :

(a) en exposant à des conditions mutagènes, de manière connue, la souche d'Aureobasidium pullulans ATCC 9348,

(b) puis en cultivant la souche de manière connue et

(c) en sélectionnant une souche d'A.-pullulans mutée (sous

la forme d'une ou de plusieurs colonies), la souche sélectionnée étant moins pigmentée que la souche de départ ou n'étant pas pigmentée.

**2.** Souche selon la revendication 1 que l'on peut obtenir en mutant par une exposition à rayonnement ultraviolet ou par un traitement chimique, en particulier par traitement avec du méthane-sulfonate d'éthyle.

**3.** Souche selon l'une des revendications 1 et 2, que l'on peut obtenir en cultivant, lors de l'étape (b), dans un intervalle de température pour lequel il ne se produit pas de multiplication cellulaire ou il ne s'en produit qu'une légère mais la formation de mélanine a encore lieu, plus particulièrement à une température située dans l'intervalle allant de 2 à 13°C, de préférence de 4 à 10°C.

**4.** Souche d'Aureobasidium pullulans DSM 3562.

**5.** Procédé pour préparer une souche d'A.-pullulans selon l'une quelconque des revendications 1 à 4, procédé caractérisé en ce qu'on effectue les étapes (a) à (c) selon la revendication 1.

**6.** Procédé selon la revendication 5 caractérisé en ce qu'on effectue les opérations selon une des revendications 2 et 3.

**7.** Application d'une souche d'A.-pullulans selon l'une quelconque des revendications 1 à 4 à la préparation de pullulane.